# EUROPEAN PATENT APPLICATION

(11) **EP 2 261 204 A1**
(43) Date of publication of application: **15.12.2010**
(21) Application number: 09722247.5
(22) Date of filing: 16.03.2009
(51) Int. Cl.: C07C 315/04, C07C 317/24, C07C 403/22

(54) **SULFONE COMPOUND AND METHOD FOR PRODUCING THE SAME**

(30) Priority: 17.03.2008 JP 2008067383; 27.10.2008 JP 2008275616; 27.10.2008 JP 2008275618; 22.01.2009 JP 2009012187
(71) Applicant: Sumitomo Chemical Company, Limited, Tokyo 104-8260 (JP)
(72) Inventor: TAKAHASHI, Toshiya, Toyonaka-shi Osaka 560-0021 (JP); SHINTAKU, Tetsuya, New York, NY 10017 (US); OZTURK, Orhan, Toyonaka-shi Osaka 561-0802 (JP); FUJII, Yohei, Minoo-shi Osaka 562-0045 (JP)
(74) Representative: Henkel, Feiler & Hänzel
(86) International application number: PCT/JP2009/055024
(87) International publication number: WO 2009/116487

(57) **Abstract**

The present invention relates to a process for producing a sulfone compound represented by formula (1), which comprising a step of obtaining a compound represented by formula (4) by reacting a compound represented by formula (3) with a chlorate or a bromate, and then resulting reaction solution with HX or X₂; a step of obtaining a compound represented by formula (5) or formula (6) by reacting a compound represented by formula (4) with a halogenating agent or M(OCOR)ₙ; or a step of obtaining a compound represented by formula (7) by reacting a compound represented by formula (5) or formula (6) with an inorganic base.

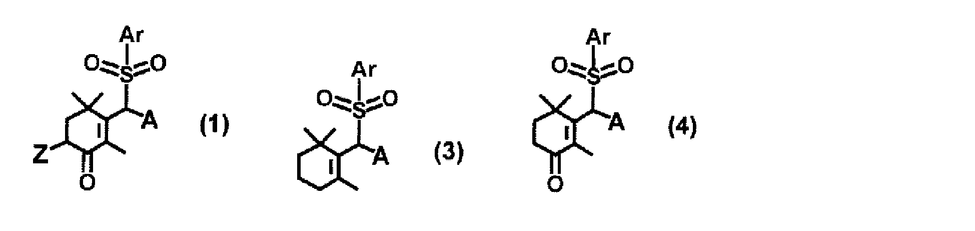

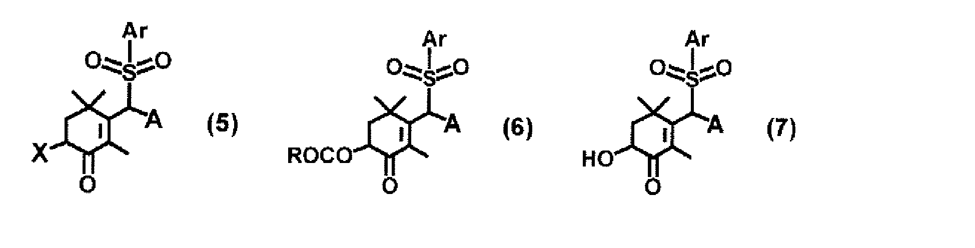

## Description

### TECHNICAL FIELD

The present invention relates to sulfone compound and a process for preparation thereof.

### BACKGROUND ART

Carotenoids such as β-carotene, canthaxanthin and astaxanthin conventionally have been used as feed additives, food coloring agents, etc. The existing processes for producing β-carotenes are described in Non-Patent Publication 1: Pure & Appl. Chem., Vol. 63, No. 1, pp.45-58, 1991, in which concretely, a process for producing a C₄₀ β-carotene from two molecules of a C₁₅ Wittig reagent and one molecule of a C₁₀ dialdehyde is described.

### DISCLOSURE OF INVENTION

### PROBLEM TO BE SOLVED BY THE INVENTION

Under such a circumstance, there has been demanded development of intermediates from which carotenoids are easily derived, and processes for producing such intermediates, superior from the viewpoints of costs of starting materials, number of productions steps, purifying step, etc.

### MEANS FOR SOLVING THE PROBLEM

As a result of the present inventors' intensive studies for solving the above-described problem, the inventors have discovered a process for preparation of a desired sulfone compound which can be produced inexpensively, safely and efficiently.

Objects and preferable embodiments of the present invention will be described below.
[1] A process for producing a compound represented by the following formula (1) in which Z is a hydrogen atom: 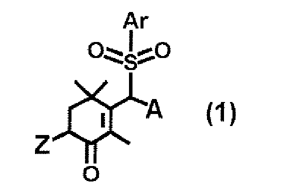 [wherein A is a hydrogen atom or a group represented by the formula (2): 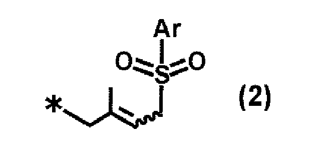 (wherein Ar is an aryl group optionally having 1 to 3 substituents, the wavy line means that the steric relation to the double bond which the wavy line is bound to is of E-form, a Z-form or a mixture of E/Z, and * shows a bonding site) and Ar means the same as defined above], comprising: a first step of reacting a compound represented by the formula (3): 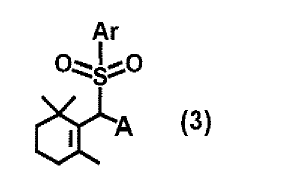 (wherein A and Ar mean the same as defined above) with a chlorate or a bromate; and
   a second step of reacting the reaction liquid obtained in the first step with either of reaction reagents: HX and X₂ wherein X is a halogen atom to give a compound represented by the formula (4): 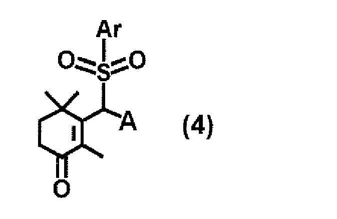 (wherein A and Ar mean the same as defined above).
[2] The process defined in the item [1], wherein the second step is performed at pH 5 or less.
[3] The process defined in the item [1] or [2], wherein the chlorate or the bromate is an alkali metal salt.
[4] The process defined in the item [3], wherein the alkali metal salt of chloric acid or bromic acid is sodium chlorate or sodium bromate.
[5] The process defined in any one of the items [1] to [4], wherein the reaction reagent used in the second step is HX wherein X is a bromine atom.
[6] The process defined in any one of the items [1] to [4], wherein the reaction reagent used in the second step is X₂ wherein X is an iodine atom.
[7] The process defined in any one of the items [1] to [6], wherein Ar is a 4-methylphenyl group.
[8] A process for producing a compound represented by the following formula (1) in which Z is a halogen atom: 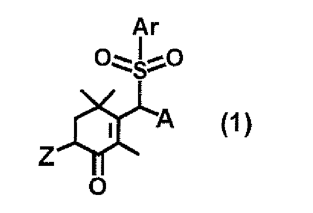 [wherein A is a hydrogen atom or a group represented by the formula (2): 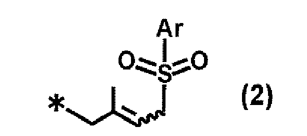 (wherein Ar is an aryl group optionally having 1 to 3 substituents, the wavy line means that the steric relation to the double bond which the wavy line is bound to is of E-form, a Z-form or a mixture of E/Z, and * shows a bonding site) and Ar means the same as defined above], comprising the step of:
   reacting a compound represented by the formula (4): 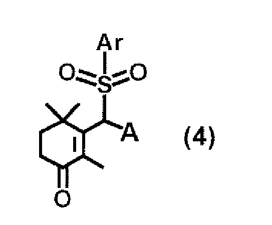 (wherein A and Ar mean the same as defined above) with a halogenating agent to give a compound represented by the formula (5): 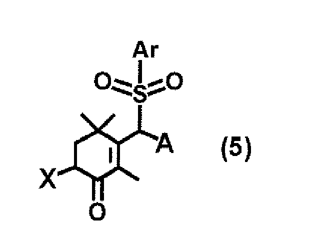
   (wherein X is a halogen atom, and A and Ar mean the same as defined above).
[9] The process defined in the item [8], wherein X is a bromine atom, and the halogenating agent is bromine, N-bromosuccinimide or 1,3-dibromo-5,5-dimethylhydantoin.
[10] The process defined in the item [8] or [9], wherein Ar is a 4-methylphenyl group.
[11] A process for producing a compound represented by the following formula (1) in which Z is an alkoxycarbonyl group: 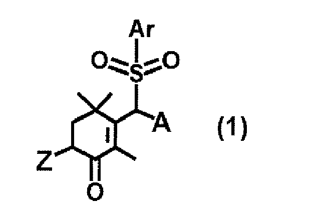 [wherein A is a hydrogen atom or a group represented by the formula (2) : 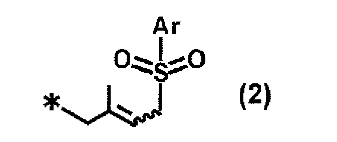 (wherein Ar is an aryl group optionally having 1 to 3 substituents, the wavy line means that the steric relation to the double bond which the wavy line is bound to is of E-form, a Z-form or a mixture of E/Z, and * shows a bonding site) and Ar means the same as defined above], comprising the step of:
   reacting a compound represented by the formula (4): 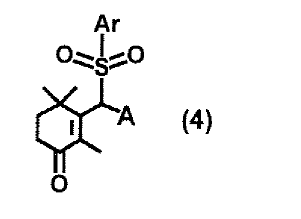 (wherein A and Ar mean the same as defined above) with a compound represented by M(OCOR)ₙ (wherein M is a metal atom, R is an alkyl group having 1 to 4 carbon atoms, and n is an integer of 2 to 4), to give a compound represented by the formula (6): 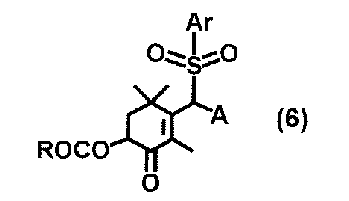 (wherein A, Ar and R mean the same as defined above).
[12] The process defined in the item [11], wherein R is a methyl group, M is manganese, and n is 3.
[13] The process defined in the item [11] or [12], wherein Ar is a 4-methylphenyl group.
[14] A process for producing a compound represented by the following formula (1) in which Z is a hydroxyl group: 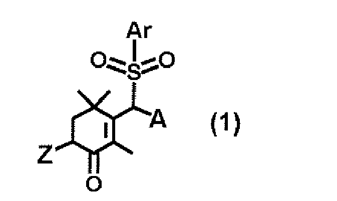 [wherein A is a hydrogen atom or a group represented by the formula (2): 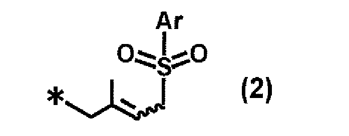 (wherein Ar is an aryl group optionally having 1 to 3 substituents, the wavy line means that the steric relation to the double bond which the wavy line is bound to is of E-form, a Z-form or a mixture of E/Z, and * shows a bonding site) and Ar means the same as defined above], comprising the step of:
   reacting a compound represented by the formula (6): 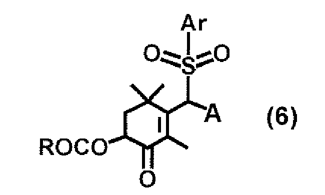 (wherein R is an alkyl group having 1 to 4 carbon atoms, and A and Ar mean the same as defined above) with an inorganic base to give a compound represented by the formula (7): 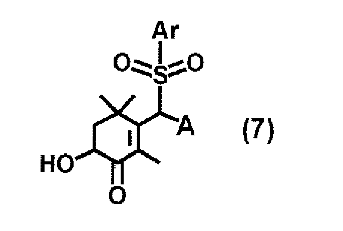 (wherein A and Ar mean the same as defined above).
[15] The process defined in the item [14], wherein the inorganic base is a hydroxide, a carbonate or a hydrogen carbonate of an alkali metal or an alkaline earth metal.
[16] The process defined in the item [14] or [15], wherein Ar is a 4-methylphenyl group.
[17] A compound represented by the formula (1): 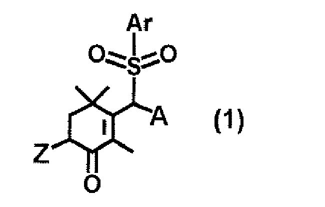 [wherein Z is a hydrogen atom, a halogen atom, an alkoxycarbonyl group having 1 to 4 carbon atoms, or a hydroxyl group; A is a hydrogen atom or a group represented by the formula (2): 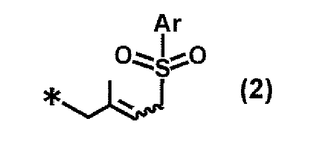 (wherein Ar is an aryl group optionally having 1 to 3 substituents, the wavy line means that the steric relation to the double bond which the wavy line is bound to is of E-form, a Z-form or a mixture of E/Z, and * shows a bonding site) and Ar means the same as defined above, provided that when A is a hydrogen atom, Z is not a halogen atom and a hydroxyl group].
[18] The compound defined in the item [17], wherein Ar is a 4-methylphenyl group.
[19] The compound defined in the item [17] or [18], wherein Z is a methoxycarbonyl group.

### EFFECT OF THE INVENTION

According to the present invention, sulfone compounds which are important intermediates for producing carotenoids can be produced by commercially advantageous processes.

### BEST MODES FOR CARRYING OUT THE INVENTION

Hereinafter, the present invention will be described in detail.

In the above-described formula (1), Z is a hydrogen atom, a halogen atom, an alkoxycarbonyl group having 1 to 4 carbon atoms or a hydroxyl group.

Examples of the halogen atom include a chlorine atom, a bromine atom and an iodine atom etc., among which a bromine atom is particularly preferable. The alkoxycarbonyl group is preferably a methoxycarbonyl group.

In the above-described formulas (1) and (3) to (7), A is a hydrogen atom or a group represented by the above-described formula (2).

In the above-described formulas (1) to (7), Ar is an aryl group optionally having 1 to 3 substituents. As the aryl group, there are exemplified a phenyl group, a naphthyl group, etc.; and as the substituents, there are exemplified a C₁-C₅ linear or branched alkyl group, a C₁-C₅ linear or branched alkoxy group, a halogen atom, a nitro group, etc. The aryl group is preferably a phenyl group; and the substitutent is preferably a C₁-C₅ linear or branched alkyl group.

Specific examples of Ar include phenyl, naphthyl, 2-methylphenyl, 3-methylphenyl, 4-methylphenyl, 4-ethylphenyl, 4-propylphenyl, 4-butylphenyl, 4-pentylphenyl, 2-methoxyphenyl, 3-methoxyphenyl, 4-methoxyphenyl, 2-fluorophenyl, 3-fluorophenyl, 4-fluorophenyl, 2-chlorophenyl, 3-chlorophenyl, 4-chlorophenyl, 2-bromophenyl, 3-bromophenyl, 4-bromophenyl, 2-iodephenyl, 3-iodephenyl, 4-iodephenyl, 2-nitorophenyl, 3-nitorophenyl, 4-nitorophenyl, 2,4-dimethylphenyl, 2,4-dimethoxyphenyl, 2,4-dichrolophenyl, 2,4,6-trimethylphenyl, 2,4,6-trichrolophenyl, etc. Preferably, Ar is an unsubstituted phenyl group or a phenyl group substituted by a C₁-C₅ linear or branched alkyl group. More preferably, Ar is a phenyl group which is substituted at its position 4 by a C₁-C₅ linear alkyl group. Particularly, Ar is 4-methylphenyl.

In the above-described formula (5), X is a halogen atom. Examples of the halogen atom include a chlorine atom, a bromine atom and an iodine atom etc., among which a bromine atom is particularly preferable.

In the following, each step of the present invention is described in detail.

The following reaction step (hereinafter referred to as "Step (A)") is an oxidation step.

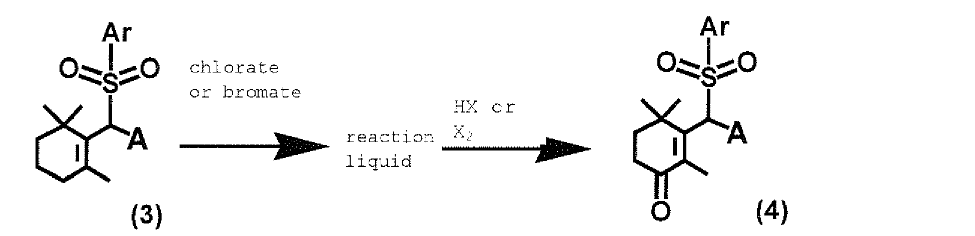

The compound represented by the formula (3) [hereinafter abbreviated as the "compound (3)"], which is a starting material in the present invention, wherein A is a hydrogen atom can be synthesized according to, for example, the method described in JP-A-2001-139542. The compound (3) wherein A is the group represented by the formula (2) can also be synthesized according to, for example, the method described in JP-A-2002-193917. Any compound thus synthesized may be used in the subsequent reaction without isolation and purification.

As the chlorate or the bromate used in the first step of Step (A), alkaline earth metal salts, alkali metal salts or ammonium salts, or free acids are used. Alkali metal salts such as sodium chlorate, potassium chlorate, sodium bromate and potassium bromate; and alkaline earth metal salts such as barium chlorate are preferable from the viewpoint of ease of availability as an industrial product, and alkali metal salts such as sodium chlorate and sodium bromate are particularly preferable from the viewpoint of reactivity and economy.

It is preferable to add the chlorate or the bromate after it is dissolved in a solvent, and examples of the solvent used include water. Of these, solvents containing no metal possibly exhibiting catalytic activity to the chlorate or the bromate are preferable, and deionized water is preferable from the viewpoint of solubility for the chlorate or the bromate, and economy.

The chlorate or the bromate is usually used in an amount 0.1 to 10 mol, preferably 0.2 to 7 mol, more preferably 0.5 to 6 mol relative to 1 mol of the compound (3).

Examples of the reaction solvent include ether solvents such as acetonitrile, tetrahydrofuran (hereinafter abbreviated as "THE"), methyl-t-butyl ether, cyclopentyl methyl ether, 1,4-dioxiane, dimethoxyethane (hereinafter abbreviated as "DME"), anisole, diglyme, triglyme and tetraglyme; and alcohol solvents such as isopropanol and t-butanol. The ether solvents are preferable, DME and THF are preferable from the viewpoint of reactivity and economy, and THF is particularly preferable from the viewpoint of operability. These solvents may be used alone or as a mixture of two or more kinds thereof.

The solvent is usually used in an amount 0.5 to 500 ml, preferably 0.7 to 200 ml, more preferably 1 to 150 ml relative to 1 g of the compound (3).

The reaction temperature is usually from -15 to 40°C, preferably from -5 to 35°C, more preferably from 0 to 35°C.

The reaction time may vary depending on the reagent used and the reaction temperature, and is usually from 0.5 to 100 hours, preferably from 3 to 100 hours, more preferably from 5 to 100 hours.

The degree of progress of the reaction can be confirmed by means of HPLC (high performance liquid chromatography).

After completion of the reaction, the reaction mixture may be used in the subsequent step without isolation and purification. Alternatively, the mixture may be isolated and purified by a usual post treatment. The purification may be performed by a usual process such as column chromatography or crystallization.

The reaction reagent used for the reaction in the second step of Step (A) is either HX or X₂ wherein X is a halogen atom. In the reaction reagent, X is preferably a bromine atom or an iodine atom. Specifically, as HX or X₂, hydrogen bromide, bromine and iodine are preferable from the viewpoint of reactivity. From the viewpoint of handling, hydrogen bromide and iodine are particularly preferable. When iodine or bromine is used, the reaction reagent may be added in a solid or liquid state without dilution, or may be dissolved in or diluted with a solvent used in the reaction and then the resulting product may be added dropwise.

When hydrogen bromide is used, the form thereof is not particularly limited so long as hydrogen bromide is contained, and an aqueous solution or an acetic acid solution of hydrogen bromide is preferable from the viewpoint of ease of availability. The aqueous solution is particularly preferable from the viewpoint of economy and ease of handling. The solutions usually have a hydrogen bromide concentration of 20 to 60%, preferably 45 to 50%. Aqueous solutions having a hydrogen bromide concentration of 45 to 50% are particularly preferable because they are generally commercially available and are inexpensive.

HX or X₂ is usually used in an amount 0.01 to 10 mol, preferably 0.05 to 5 mol relative to 1 mol of the compound (3).

The kind and amount of the solvent, the reaction temperature and the reaction time used in the second step may be similar to those in the first step.

The second step is preferably performed at pH 5 or less. In particular, when X₂ is used in the reaction, the pH of the reaction system is preferably within a range of 1 to 4 during the reaction. In order to adjust the pH value within this range, sulfuric acid, nitric acid, acetic acid or a buffer mixture is used. Sulfuric acid, nitric acid and acetic acid are preferable from the viewpoint of ease of availability as an industrial product, and sulfuric acid is particularly preferable from the viewpoint of reactivity and economy.

After completion of the reaction, the reaction mixture may be used in the subsequent step without isolation and purification. Alternatively, the reaction mixture may be isolated and purified by a usual post treatment such as column chromatography or crystallization.

Usually, to a reaction vessel are added the solvent, the compound (3), and the aqueous chlorate or bromate solution and the first step is advanced, and then the solution of HX or X₂ (after the pH of the reaction system is adjusted within an adequate range) is added dropwise and the second step is advanced. When the aqueous chlorate or bromate solution and the solution of HX or X₂ is added dropwise, required equivalents of the solution may be added at once, or several aliquots may be separately added.

The following reaction step [hereinafter referred to as "Step (B)"] is a halogenation step.

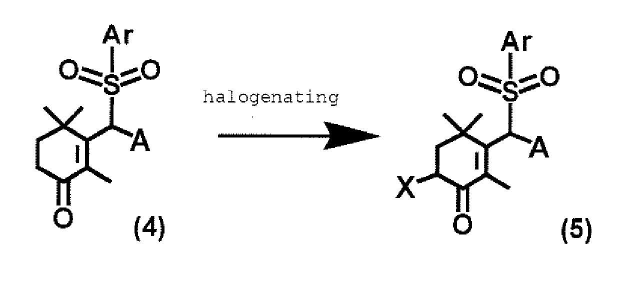

Examples of the halogenating agent used in Step (B) include bromine, N-bromosuccinimide, 1,3-dibromo-5,5-dimethylhydantoin, and copper (II) bromide. Bromine, N-bromosuccinimide, and 1,3-dibromo-5,5-dimethylhydantoin are preferable from the viewpoint of operability, and bromine is more preferable from the viewpoint of economy.

The halogenating agent is usually used in an amount 0.48 to 2 mol, preferably 0.5 to 1.5 mol, more preferably 0.55 to 1.2 mol relative to 1 mol of the compound represented by the formula (4) [hereinafter abbreviated as the "compound (4)"]. When the halogenating agent is in a solid state, the agent may be added as it is, or may be added in a solution state after dissolving the agent in a solvent used in the reaction. When the halogenating agent is in a liquid state, the agent may be added dropwise after it is diluted with a solvent used in the reaction.

Examples of the reaction solvent used in Step (B) include hydrocarbon solvents such as toluene and chlorobenzene; ester solvents such as methyl acetate, ethyl acetate, butyl acetate and isopropyl acetate; and alcohol solvents such as methanol, ethanol, n-propanol, 2-propanol, n-butanol and t-butanol. Chlorobenzene, ethyl acetate and methanol may be exemplified from the viewpoint of reactivity and economy, and ethyl acetate and chlorobenzene are particularly preferable from the viewpoint of operability.

The solvent is usually used in an amount 0.5 to 500 ml, preferably 0.7 to 200 ml, more preferably 1 to 150 ml relative to 1 g of the compound (4).

The reaction temperature is usually from 0 to 100°C, preferably from 0 to 30°C, more preferably from 0 to 20°C.

After completion of the reaction, the reaction mixture may be used in the subsequent step without isolation and purification. Also, the resulting compound represented by the formula (5) [hereinafter abbreviated as the "compound (5)"] may be isolated and purified by a usual post treatment such as column chromatography or crystallization.

The following reaction step [hereinafter referred to as "Step (C)"] is an esterification step.

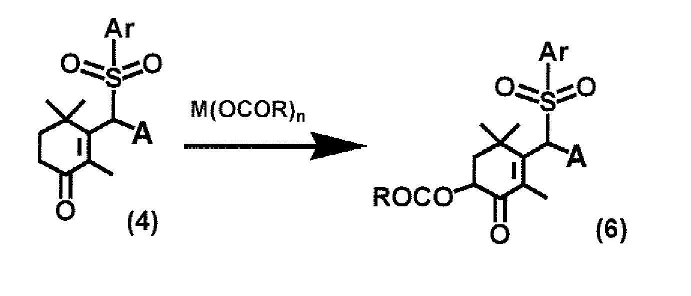

The reagent used in Step (C) is represented by M(OCOR)ₙ wherein M is a metal atom, R is an alkyl group having 1 to 4 carbon atoms, and n is an integer of 2 to 4. Examples of the metal atom include manganese and lead, etc. A preferable metal atom is manganese, and a preferable alkyl group is an alkyl group having 1 to 2 carbon atoms.

Specific examples of the reagent used include manganese triacetate and lead tetraacetate, etc. When the reagent is a hydrate, it is preferably subjected to the reaction after being dehydrated by azeotropy with an organic solvent, or the like. The reagent is usually used in an amount 0.1 to 20 mol, preferably 0.5 to 10 mol, more preferably 1 to 7 mol relative to 1 mol of the compound (4).

Examples of the reaction solvent used in Step (C) include acetic acid, and a mixed solvent of an organic solvent and acetic acid. As the organic solvent, hydrocarbon solvents are preferable, and examples thereof include toluene, xylene, hexane, cyclohexane, and heptane, etc.

The reaction temperature is usually within a range of 50°C to the boiling point of the solvent used, preferably within a range of 80 to 120°C.

The reaction time is usually arbitrarily selected from a range of 1 to 48 hours, and it depends on the kind and amount of the reagent used.

After completion of the reaction, the reaction mixture may be used in the subsequent step only after filtering the reaction reagent off without isolation and purification. Alternatively, the resulting compound represented by the formula (6) [hereinafter abbreviated as the "compound (6)"] may be isolated and purified by a usual post treatment.

The purification may be performed by a usual process such as column chromatography or crystallization.

The following reaction step [hereinafter referred to as "Step (D)"] is a hydrolysis step.

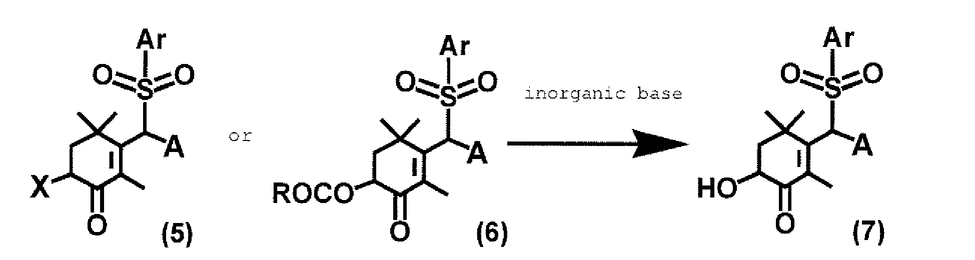

As the inorganic base used in Step (D), a hydroxide, a carbonate or a hydrogen carbonate of an alkali metal or an alkaline earth metal is preferable. Specific examples of the inorganic base include sodium carbonate, potassium carbonate, calcium carbonate, sodium hydroxide, potassium hydroxide, calcium hydroxide, sodium hydrogen carbonate, potassium hydrogen carbonate, and calcium hydrogen carbonate.

It is preferable that the base used is dissolved in a solvent, and then the resulting mixture is added. As the solvent used, solvents such as water are exemplified from the viewpoint of economy. Usually, the solvent is not particularly limited so long as it is water generally used. The base is usually used in an amount 0.1 to 100 mol, preferably 0.5 to 30 mol relative to 1 mol of the compound (5) or (6).

The reaction does not necessarily require to use a phase-transfer catalyst, but addition of a phase-transfer catalyst may accelerate the reaction.

Examples of the phase transfer catalyst include quaternary ammonium salts, quaternary phosphonium salts, and sulfonium salts, and quaternary ammonium salts are preferable.

Examples of the quaternary ammonium salt include tetramethylammonium chloride, tetraethylammonium chloride, tetrapropylammonium chloride, tetrabutylammonium chloride, tetrapentylammonium chloride, tetrahexylammonium chloride, tetraheptylammonium chloride, tetraoctylammonium chloride, trioctylmethylammonium chloride, tetradecylammonium chloride, tridecylmethylammonium chloride, didecyldimethylammonium chloride, tetradodecylammonium chloride, tridodecylmethylammonium chloride, didodecyldimethylammonium chloride, dodecyltrimethylammonium chloride, dodecyltriethylammonium chloride, tetradecyltrimethylammonium chloride, tetrahexadecylammonium chloride, hexadecyltrimethylammonium chloride, hexadecyldimethylethylammonium chloride, tetraoctadecylammonium chloride, octadecyltrimethylammonium chloride, octadecyltriethylammonium chloride, benzyltrimethylammonium chloride, benzyltriethylammonium chloride, benzyltributhylammonium chloride, 1-methylpyridinium chloride, 1-hexadecylpyridinium chloride, 1,4-dimethylpyridinium chloride and trimethlycyclopropylammonium chloride; or compounds which are bromide salts, iodide salts or hydrogensulfates corresponding to these chloride salts.

Examples of the quaternary phosphonium salt include tributylmethylphosphonium chloride, triethylmethylphosphonium chloride, methyltriphenoxyphosphonium chloride, butyltriphenylphosphonium chloride, tetrabutylphosphonium chloride, benzyltriphenylphosphonium chloride, tetraoctylphosphonium chloride, hexadecyltrimethylphosphonium chloride, hexadecyltributylphosphonium chloride, hexadecyldimethylethylphosphonium chloride and tetraphenylphosphonium chloride; or compounds which are bromide salts or iodide salts corresponding to these chloride salts.

Examples of the sulfonium salt include benzylmethylethylsulfonium chloride, benzyldimethylsulfonium chloride, benzyldiethylsulfonium chloride, dibutylmethylsulfonium chloride, trimethylsulfonium chloride, triethylsulfonium chloride and tributylsulfonium chloride; or compounds which are bromide salts or iodide salts corresponding to these chloride salts.

The amount of such a phase-transfer catalyst to be used is usually from 0.005 to 2 times by mole, preferably from about 0.01 to 0.5 times by mole relative to 1 mol of the compound (5) or (6).

Examples of the solvent used in Step (D) include lower alcohols such as methanol, ethanol, 2-propanol and t-butanol; ether solvents such as tetrahydrofuran, cyclopentyl methyl ether, 1,4-dioxane, methyl-t-butyl ether, ethylene glycol dimethyl ether, diglyme and triglyme; aprotic polar solvents such as N,N-dimethyl formamide and N,N-dimethyl acetamide; and hydrocarbon solvents such as toluene, xylene, benzene, heptane, pentane, monochlorobenzene and dichlorobenzene.

The solvent is usually used in an amount 0.5 to 500 ml, preferably 0.7 to 200 ml, more preferably 1 to 150 ml relative to 1 g of the compound (5) or (6).

The reaction temperature is usually from -5 to 20°C, preferably from 0 to 15°C, more preferably from 0 to 10°C, while the base is added dropwise. After completion of the addition of the base, the temperature is from 0 to 60°C, preferably from 0 to 50°C, more preferably from 0 to 40°C.

The addition process in Step (D) is not particularly limited, but it is preferable to add the solvent and the compound (5) or (6) to a reaction vessel, and then to add the solution of the inorganic base dropwise thereto, from the viewpoint of safety and operability.

The reaction time may vary depending on the reagent used and the reaction temperature, and it is usually from 0.5 to 200 hours, preferably from 1 to 50 hours.

The degree of progress of the reaction can be confirmed by means of HPLC (liquid chromatography).

After completion of the reaction, the compound represented by the formula (7) [hereinafter abbreviated as the "compound (7)"] may be isolated and purified by a usual post treatment. The purification may be performed by a usual method such as column chromatography or crystallization.

The compounds (4) and (7) of the present invention can serve as important intermediates of a carotenoid (canthaxanthin or astaxanthin). That is, by reacting the compounds (4) or (7) with an allyl halide having 10 carbon atoms under basic conditions, a carotenoid (canthaxanthin or astaxanthin) can be synthesized.

### EXAMPLES

The present invention will be described in more detail by way of Examples, which however should not be construed as limiting the scope of the present invention in any way.

The chemical formulas of the compounds used in Examples are shown below.

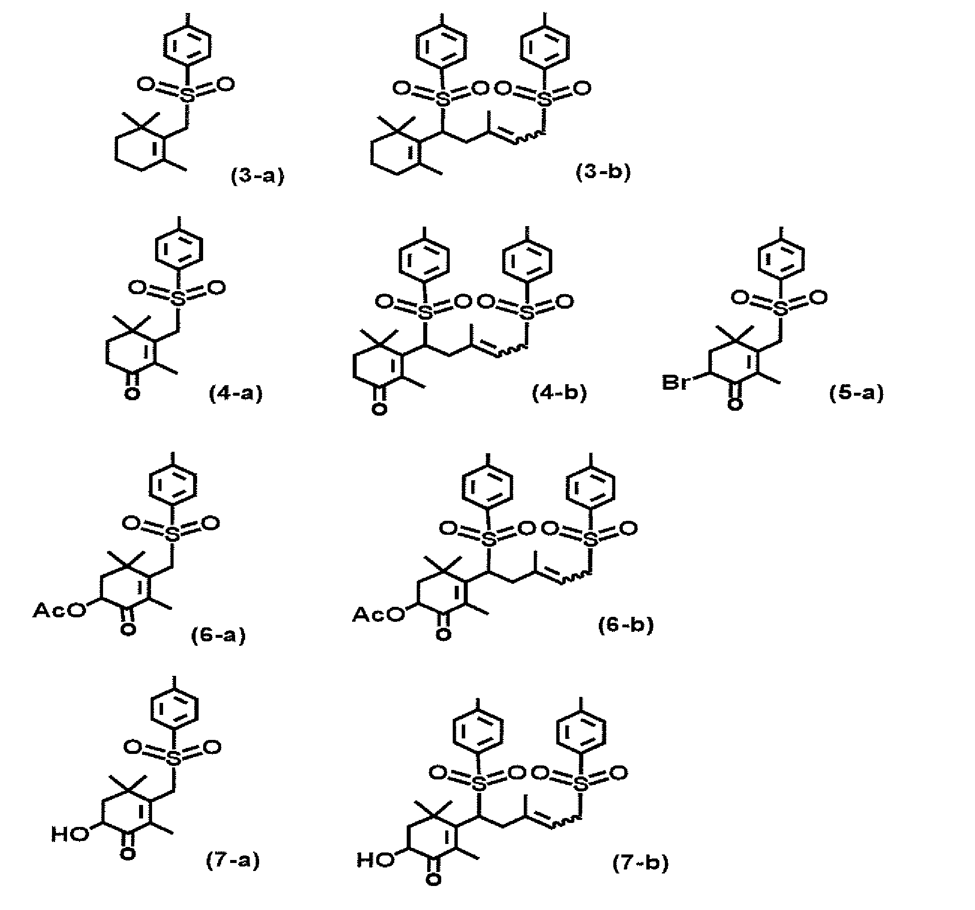

### (Example 1)

To a 100 ml two-necked flask were added a compound (3-a) (1.30 g, 4.45 mol) and ethyl acetate (50 ml), and the mixture was stirred under ice-cooling. Sodium bromate (1.34 g, 8.89 mol) was dissolved in deionized water (30 ml) and the resulting solution was added thereto. Next, hydrobromic acid (a 30% acetic acid solution, 240 mg, 0.89 mol) was added dropwise thereto at 10°C or less, and the mixture was stirred for 2 hours. After that, the bath was removed, and the mixture was stirred at a temperature within a range of 20 to 30°C for 7 hours. The reaction mixture was washed twice with 20% brine (50 ml). The organic layer was washed twice with a 10% aqueous sodium bisulfite solution (50 ml), and dried over magnesium sulfate. After concentration under reduced pressure, the residue was purified by column chromatography (SiO₂, hexane-ethyl acetate:5:1) to obtain a compound (4-a)(0.95 g). Yield: 69.7%, purity: 99.0%.

Analysis data:
¹H-NMR (500MHz,CDCl₃): δ = 7.82 (d, J=8.4Hz, 2H), 7.38 (d, J=7.7Hz, 2H), 4.16 (S, 2H), 2.55 (t, J=6.9Hz, 2H), 2.46 (S,3H), 1.89 (t, J=6.9Hz, 2H), 1.78 (S, 3H), 1.25 (S, 6H). ¹³C-NMR (100MHz, CDCl₃) : 149.9, 144.8, 138.0, 137.7, 129.9, 127.5, 58.7,37.1, 35.5, 34.0, 27.1, 21.4, 13.2.

### (Example 2)

To a 500 ml four-necked flask were added a compound (3-a) (30.0 g, 0.10 mol) and ethyl acetate (300 ml), and the mixture was stirred under ice-cooling. Sodium bromate (7.74 g, 0.05 mol) was dissolved in deionized water (30 ml) and the resulting solution was added thereto. Next, hydrobromic acid (a 48% aqueous solution, 8.65 g, 0.05 mol) was added dropwise thereto at 10°C or less, and the mixture was stirred for 2 hours. After that, the bath was removed, and the mixture was stirred at a temperature within a range of 20 to 30°C for 7 hours. The reaction mixture was washed twice with 20% brine (200 ml). The organic layer was washed twice with a 10% aqueous sodium bisulfite solution (100 ml), and dried over magnesium sulfate. The solvent was distilled off by concentration under reduced pressure, and then methanol (50 ml) was added thereto, and the mixture was heated to 45°C to dissolve the residue. After the solution was gradually cooled to 25°C, deionized water (10 ml) was added dropwise thereto, and the mixture was stirred at a temperature within a range of 15 to 20°C for 2 hours, and filtered to obtain a compound (4-a) (19.9 g). Yield: 63.2%, purity: 99.0%.

### (Example 3)

To a 500 ml four-necked flask were added a compound (3-a) (30.0 g, 0.10 mol) and ethyl acetate (150 ml), and the mixture was stirred under ice-cooling. Sodium bromate (7.74 g, 0.05 mol) was dissolved in deionized water (30 ml) and the resulting solution was added thereto. Next, hydrobromic acid (a 48% aqueous solution, 5.19 g, 0.03 mol) was added dropwise thereto at 10°C or less, and the mixture was stirred for 2 hours. After that, the bath was removed, and the mixture was stirred at a temperature within a range of 20 to 30°C for 7 hours. The reaction mixture was washed twice with 20% brine (150 ml). The organic layer was washed twice with a 10% aqueous sodium bisulfite solution (150 ml), and dried over magnesium sulfate. After the solvent was distilled off by concentration under reduced pressure, the residue was used in subsequent reaction.

### (Example 4)

To a 500 ml four-necked flask were added the residue obtained from Example 3 and ethyl acetate (150 ml), and the mixture was stirred under ice-cooling. Bromine (14.8 g, 0.093 mol) was added dropwise thereto at 10°C or less. After the mixture was stirred for 2 hours, the reaction mass was added dropwise to a 10% aqueous sodium bisulfite solution (150 ml) and washed. The mixture was washed twice with sodium bicarbonate water (120 ml). The organic layer was dried over magnesium sulfate, and the solvent was distilled off by concentration under reduced pressure. Ethyl acetate (80 ml) was added to the residue, and the mixture was heated to 45°C to dissolve the residue. After the solution was gradually cooled to 25°C, hexane (120 ml) was added dropwise thereto. After the solution was cooled to 15°C and stirred for 2 hours, and filtered to obtain a compound (5-a)(20.6 g). Yield: 52.1%(two-step), purity: 99.0%.

Analysis data:
¹H-NMR (500MHz,CDCl₃): δ = 7.82 (d, J=8.4Hz, 2H), 7.40 (d, J=8.4Hz, 2H), 4.93 (dd, J=13.0Hz, 6.2Hz, 1H), 4.18 (d, J=14.5Hz, 1H), 4.09 (d, J=13.8Hz, 1H), 2.47 (S, 3H), 2.46-2.39 (m, 2H) ,1.88 (S, 3H), 1.33 (S, 3H), 1.29 (S, 3H). ¹³C-NMR (100MHz, CDCl₃) : 191.0, 150.2, 145.2, 138.0, 136.6, 130.1, 127.6, 58.8, 49.3, 48.8, 38.6, 28.9, 26.0, 21.6, 14.4.

### (Example 5)

A 30 ml two-necked flask was charged with a compound (4-a) (20 mg, 0.07 mmol) and methanol (2 ml), and the mixture was stirred under the room temperature. N-bromosuccinimide (12.8 mg, 0.07 mmol) was added thereto, and the mixture was stirred at a temperature within a range of 20 to 30°C for 12 hours. This reaction solution was analyzed by LC. As a result, it was confirmed that a compound (5-a) was produced at a yield of 95.9% (area percentage).

Conditions for LC analysis:
SUMIPAX ODS A-210EC (3.0 mmϕ × 150 mm, 5µm)
Mobile phase: solution A: 0.05% TFA/water, and
   solution B: methanol
   flow rate: 0.5 mL/min
Column temperature: 40°C
Solution B: 50% (15 min) → (30 min) → 80%

### (Example 6)

A 30 ml two-necked flask was charged with a compound (4-a) (20 mg, 0.07 mol) and methanol (2 ml), and the mixture was stirred under the room temperature. 1,3-dibromo-5,5-dimethylhydantoin (11.8 mg, 0.04 mmol) was added thereto. The mixture was stirred at a temperature within a range of 20 to 30°C for 12 hours. This reaction solution was analyzed by LC. As a result, it was confirmed that a compound (5-a) was produced at a yield of 57.9% (area percentage) (the condition for analysis was the same as that in Example 5).

### (Example 7)

To a 500 ml four-necked flask were added a compound (5-a) (1.05 g, 2.7 mmol) and N,N-dimethyl acetamide (90 ml), and the mixture was stirred under ice-cooling. Potassium carbonate (3.50 g, 25.3 mmol) was dissolved in deionized water (20 ml) and added dropwise at 10°C or less over 3 hours. After that, the bath was removed, and the mixture was stirred at a temperature within a range of 20 to 30°C for 24 hours. To the reaction mass was added toluene (500 ml), and the mixture was concentrated under reduced pressure. To the residue was added ethyl acetate (300 ml) to dissolve the residue, and then the solution was washed three times with 20% brine (150 ml) and was dried over magnesium sulfate. After concentration under reduced pressure, the residue was purified by column chromatography (SiO₂, hexane-ethyl acetate: 4 : 1) to obtain a compound (7-a) (244 mg). Yield: 27.9%, purity: 99.0%.

Analysis data:
¹H-NMR (500MHz,CDCl₃): δ = 7.73 (d, J=8.4Hz, 2H), 7.30 (d, J=8.4Hz, 2H), 4.30 (dd, J=13.8Hz, 6.1Hz, 2H), 4.10 (d, J=13.8Hz, 1H), 4.00 (d, J=13.8Hz, 1H), 3.58 (S, 1H), 2.37 (S, 3H), 2.07 (dd, J=13.0Hz, 5.3Hz, 1H), 1.82 (S, 3H), 1.82-1.74 (m, 1H), 1.23 (S, 3H), 1.14 (S, 6H). ¹³C-NMR (100MHz, CDCl₃) : 199.6, 150.7, 145.0, 138.1, 135.2, 129.9, 127.4, 69.2, 58.5, 45.3, 36.8, 29.6, 25.5, 21.4, 13.6.

### (Example 8)

A flask was charged with Mn(OAc)₃·2H₂O (2.5 MR, 4.6 g, 17.1 mmol) and dehydrated toluene(20 ml), and the mixture was concentrated under reduced pressure to distill water off. Subsequently, acetic acid (20 ml) was added at room temperature, and the mixture was stirred for 30 minutes under a nitrogen atmosphere. Next, a compound (4-a) (1.0 MR, 2.0 g, 6.84 mmol) was added at room temperature, and the temperature was elevated to 80 to 85°C. When the internal temperature reached 80°C, the reaction mixture turned into black. When the mixture was stirred at the same temperature for another 7 hours, the solution turned transparent and yellow. This phenomenon was caused by a Mn (II) compound as a white solid deposited with the advance of the reaction. After completion of the reaction, the reaction mixture was observed by HPLC or TLC, and a post treatment was performed when the LC (area percentage) of the starting compound (4-a) reached about 1%. The reaction mixture was cooled to room temperature, and was diluted with ethyl acetate (20 ml). The solid matter was filtered off, and the solution was concentrated to obtain a light brown oily compound (6-a). The product was analyzed by HPLC, and it was found that the product contained 87.1% of the compound (6-a) and 0.9% (LC area percentage) of the compound (4-a).

The structure of compound (4-a) was analyzed by NMR and MS. Analysis data:
¹H-NMR (CDCl₃, δ): 7.8 (2H, d, j= 7.6), 7.4 (2H d, j= 8.4), 5.5 (1H, dd, j= 5.3, 13.7), 4.1 (2H, dd, j=13.7), 2.5 (3H, s), 2.2 (3H, s), 2.01-2.07-2.15 (2H, m), 1.8 (3H, s), 1.4 (3H, s), 1.3 (3H, s).
FD-MS: 365 M+H.

### (Example 9)

A flask was charged with Mn(OAc)₃·2H₂O (1.25 MR, 2.3 g, 8.55 mmol) and dehydrated toluene (20 ml), and the contents were dehydrated under refluxing with a Dean-Stark trap. After the reaction mixture was cooled to room temperature, the compound (4-a) (1.0 MR, 2.0 g, 6.84 mmol) was added thereto at room temperature, the temperature of the mixture was elevated to 125°C, and the mixture was stirred for 7 hours. When the reaction was confirmed by TLC, it was found that the reaction mixture contained the starting compound as a main component. Therefore, Mn(OAc)₃·2H₂O (0.25 MR, 0.46 g, 1.71 mmol) was added additionally, and the mixture was reacted at the same temperature for 7 hours. After that, the reaction mixture was cooled to room temperature and diluted with ethyl acetate (20 ml). Next, the Mn (II) compound was filtered off, and the solution was concentrated to obtain a crude product containing the light brown oily compound (6-a). The crude product was purified by silica gel chromatography (ethyl acetate/hexane = 3/7 to 1/1) to obtain 0.95 g (yield: 38%) of the compound (6-a).

### (Example 10)

The compound (6-a) (1.0 MR, 0.1 g, 0.27 mmol) was dissolved in dimethyl formamide (10 ml), and the mixture was cooled to 0°C. Next, a solution of K₂CO₃ (15.0 MR, 0.56 g, 4.05 mol) in water (10 ml) was slowly added dropwise thereto at the same temperature. After that, the reaction mixture was allowed to naturally warm to room temperature. After the mixture was stirred at room temperature for 3 hours, the mixture was analyzed by HPLC. It was found that the product contained 73% of the compound (7-a) and 2% (LC area percentage) of the compound (6-a).

### (Example 11)

The compound (6-a) (1.0 MR, 0.1 g, 0.27 mmol) was dissolved in tetrahydrofuran (10 ml), and the mixture was cooled to 0°C. Next, a solution of K₂CO₃ (50.0 MR, 1.86 g, 13.5 mol) in water (10 ml) was slowly added dropwise thereto at the same temperature. After that, the reaction mixture was allowed to naturally warm to room temperature. After the mixture was stirred at room temperature for 7 days, the mixture was analyzed by HPLC. It was found that the product contained 81% of the compound (7-a) and 4% (LC area percentage) of the compound (6-a).

### (Example 12)

The compound (6-a) (1.0 MR, 0.1 g, 0.27 mmol) was dissolved in ethanol (10 ml), and the mixture was cooled to 0°C. Next, a solution of NaOH (2.0 MR, 0.22 g, 0.54 mmol) in water (1 ml) was slowly added dropwise thereto at the same temperature. After that, the reaction mixture was allowed to naturally warm to room temperature. After the mixture was stirred at room temperature for 3 hours, the mixture was analyzed by HPLC. It was found that the starting compound dissipated and the product contained 62% (LC area percentage) of the compound (7-a).

### (Example 13)

The compound (6-a) (1.0 MR, 0.1 g, 0.27 mmol) was dissolved in tetrahydrofuran (10 ml), and the mixture was cooled to 0°C. Next, a solution of NaOH (2.4 MR, 0.26 g, 0.65 mmol) in water (1 ml) was slowly added dropwise thereto at the same temperature. After that, the reaction mixture was allowed to naturally warm to room temperature. After the mixture was stirred for 22 hours, the mixture was analyzed by HPLC. It was found that the product contained 87% (LC area percentage) of the compound (7-a) and 9% of the compound (6-a).

### (Example 14)

Under a nitrogen atmosphere, a compound (3-b) (1.0 MR, 5 g, 9.71 mmol) was dissolved in tetrahydrofuran (400 ml), and the mixture was cooled to 4°C. Then, a solution of sodium bromate (NaBrO₃, 2.0 MR, 2.93 g, 19.4 mmol) in water (60 ml) was slowly added dropwise thereto at the same temperature. Subsequently, a 48% aqueous hydrogen bromide solution (HBr, 0.5 MR, 0.82 g, 4.86 mmol) was slowly added dropwise at the same temperature. After that, the reaction mixture was allowed to naturally warm to 25 to 30°C, and was stirred at 25 to 30°C overnight. Next, in the same manner as above, addition of a solution of sodium bromate (NaBrO₃, 0.5 MR, 733 mg, 2.43 mmol) in water (3 ml) and a 48% aqueous hydrogen bromide solution (HBr, 0.5 MR, 0.82 g, 4.86 mmol) was performed twice a day for 3 days, and the resulting mixture was stirred [sodium bromate (NaBrO₃, 5.0 MR, 7.30 g, 48.6 mol), and a 48% aqueous hydrogen bromide solution (HBr, 3.5 MR, 5.74 g, 34.0 mmol) in total]. After the resulting mixture was stirred for 94 hours from the starting of the reaction, the reaction yield thereof was calculated by using HPLC (internal standard method), and it was found that the yield of the compound (4-b) was 85%.

The structure of compound (4-b) was analyzed by NMR and MS. Analysis data:
¹H-NMR (500 MHz, CDCl₃): 7.74 (d, J=8.4Hz, 2H), 7.69 (d, J=8.4Hz, 2H), 7.33-7.36 (m, 4H), 5.16 (dd, J=8.0Hz, 1H), 4.08 (m, 1H), 3.61-3.71 (m, 2H), 3.05 (dd, J=6.9Hz, 1H), 2.65 (dd, J=6.9Hz, 1H), 2.46 (s, 3H), 2.45 (s, 3H), 2.13 (s, 3H), 1.91-1.97 (m, 1H), 1.73-1.81 (m, 1H), 1.25 (s, 3H), 1.21 (s, 3H), 0.96 (s, 3H). FD-MS: 529 (M+).

### (Example 15)

Under a nitrogen atmosphere, a compound (3-b) (1.0 MR, 250 mg, 0.486 mol) was dissolved in tetrahydrofuran (20 ml), and the mixture was cooled to 4°C. Then, a solution of sodium bromate (NaBrO₃, 5.0 MR, 365 mg, 2.43 mmol) in water (3 ml) was slowly added dropwise thereto at the same temperature. Subsequently, a 48% aqueous hydrogen bromide solution (HBr, 3.5 MR, 287 mg, 1.70 mmol) was slowly added dropwise at the same temperature. After that, the reaction mixture was allowed to naturally warm to 25 to 30°C, and was stirred at 25 to 30°C for 24 hours. The reaction yield thereof was calculated by using HPLC (internal standard method), and it was found that the yield of the compound (4-b) was 62%.

### (Example 16)

Under a nitrogen atmosphere, a compound (3-b) (1.0 MR, 250 mg, 0.486 mmol) was dissolved in tetrahydrofuran (20 ml), and the mixture was cooled to 4°C. Then, a solution of sodium bromate (NaBrO₃, 2.0 MR, 146 mg, 0.972 mmol) in water (1 ml) was slowly added dropwise thereto at the same temperature. Subsequently, a 48% aqueous hydrogen bromide solution (HBr, 0.5 MR, 41 mg, 0.243 mmol) was slowly added dropwise at the same temperature. After that, the reaction mixture was allowed to naturally warm to 25 to 30°C, and was stirred at 25 to 30°C for 1 hour. Next, in the same manner as above, addition of a solution of sodium bromate (NaBrO₃, 0.5 MR, 36.5 mg, 0.243 mmol) in water (250 µl) and a 48% aqueous hydrogen bromide solution (HBr, 0.5 MR, 41 mg, 0.243 mmol) was performed six times every hour, and the resulting mixture was stirred [sodium bromate (NaBrO₃, 5.0 MR, 365 mg, 2.43 mmol), and a 48% aqueous hydrogen bromide solution (HBr, 3.5 MR, 287 mg, 1.7 mmol) in total]. After the resulting mixture was stirred for 48 hours from the starting of the reaction, the reaction yield thereof was calculated by using HPLC (internal standard method), and it was found that the yield of the compound (4-b) was 51%.

### (Example 17)

The reaction was carried out in the same manners as in Example 14, except that dimethoxyethane was used as substitute for tetrahydrofuran. After the resulting mixture was stirred for 94 hours from the starting of the reaction, the reaction yield thereof was calculated by using HPLC (internal standard method), and it was found that the yield of the compound (4-b) was 44%.

### (Example 18)

Under a nitrogen atmosphere, the compound (3-b) (1.0 MR, 3 g, 5.83 mmol) was dissolved in tetrahydrofuran (18 ml) at 25 to 30°C. Then, a solution of sodium bromate (2.0 MR, 1.76 g, 11.7 mmol) in water (5 ml) was slowly added dropwise at the same temperature. Subsequently, sulfuric acid was added to adjust the pH of the reaction system to 1 to 2. Further, a solution of iodine (0.02 MR, 30 mg, 11.7 µmol) in THF (2 ml) was added dropwise at the same temperature, and the mixture was stirred at 25 to 30°C overnight. Next, in the same manner as above, a solution of sodium bromate (1.0 MR, 880 mg, 5.83 mol) in water (2 ml) was added, to which sulfuric acid was added to adjust the pH of the reaction system to 1 to 2, and then a solution of iodine (0.02 MR, 30 mg, 11.7 µmol) in THF (10 ml) was added, and the mixture was stirred at 25 to 30°C for 8 hours. Further, in the same manner as above, a solution of sodium bromate (0.5 MR, 440 mg, 2.92 mmol) in water (2 ml) was added, to which sulfuric acid was added to adjust the pH the reaction system to 1 to 2, and then a solution of iodine (0.02 MR, 30 mg, 11.7 µmol) in THF (10 ml) was further added, and the mixture was stirred at 25 to 30°C overnight. Finally, in the same manner as above, a solution of sodium bromate (0.5 MR, 440 mg, 2.92 mmol) in water (2 ml) was added, to which sulfuric acid was added to adjust the pH of the reaction system to 1 to 2, and then a solution of iodine (0.02 MR, 30 mg, 11.7 µmol) in THF (10 ml) was further added, and the mixture was stirred at 25 to 30°C [sodium bromate (4.0 MR, 3.52 g, 23.3 mol), and iodine (0.08 MR, 120 mg, 466 µmol) in total]. After stirring for 51 hours from the starting of the reaction, the reaction yield of the compound (4-b) was calculated by using HPLC (internal standard method), and it was found that the yield thereof was 68%.

### (Example 19)

Mn(OAc)₃·2H₂O (2.5 MR, 1.27 g, 4.73 mmol) and toluene (20 ml) were added to a flask, and the contents were concentrated under reduced pressure to distill the hydrate off, and the resulting product was used as it is. To dehydrated Mn(OAc)₃ was added acetic acid (20 ml) at room temperature, and the mixture was stirred under a nitrogen atmosphere for 30 minutes. Next, the compound (4-b) (1.0 MR, 1.0 g, 1.89 mmol) was added at room temperature, and the temperature was elevated to 80 to 85°C. When the internal temperature reached 80°C, the reaction mixture turned into black. When the mixture was stirred at the same temperature for another 10 hours, the solution turned transparent and yellow. This phenomenon was caused by a Mn (II) compound as a white solid deposited with the advance of the reaction. After completion of the reaction, the reaction product was observed by HPLC or TLC, and when the LC of the starting compound (4-b) reached about 6% (area percentage), a post treatment was performed. The reaction mixture was cooled to room temperature, and diluted with ethyl acetate (20 ml). The solid matter was filtered off, and the solution was concentrated to obtain a white compound (6-b). The crude product was purified by silica gel column chromatography to isolate the starting compound (4-b) and the compound (6-b) (an isomer mixture) at yields of 6% and 70%, respectively.

The structure of compound (6-b) was analyzed by NMR and MS. Analysis data:
¹H-NMR (CDCl₃, δ): 7.69-7.74 (4H, m), 7.34-7.36 (4H, m), 5.42-5.51 (1H, m), 5.17-5.23 (1H, m), 4.02-4.05 (1H, m), 3.61-3.73 (2H, m), 2.57-3.17 (2H, m), 2.45 (3H, s), 2.46 (3H, s), 2.14-2.18 (6H, m), 1.93-2.12 (2H, m), 1.24-1.32 (6H, 2xs), 1.01-1.09 (3H, 2xs). FD-MS: 587 (M+H).

### (Example 20)

Mn(OAc)₃·2H₂O (2.5 MR, 3.8 g, 14.2 mmol) and toluene (40 ml) were added to a flask, and the contents were concentrated under reduced pressure to distill the hydrate off, and the resulting product was used as it is. To dehydrated Mn(OAc)₃ was added acetic acid (30 ml) at room temperature, and the mixture was stirred under a nitrogen atmosphere for 30 minutes. Next, the compound (4-b) (1.0 MR, 3.0 g, 5.67 mmol) was added at room temperature, and the temperature was elevated to 80 to 85°C. When the internal temperature reached 80°C, the reaction mixture turned into black. When the mixture was stirred at the same temperature for another 9 hours, the solution turned transparent and yellow. This phenomenon was caused by a Mn (II) compound as a white solid deposited with the advance of the reaction. After completion of the reaction, the reaction product was observed by HPLC or TLC, and when the LC of the starting compound (4-b) reached about 5 to 6% (area percentage), a post treatment was performed. The reaction mixture was cooled to room temperature, and diluted with ethyl acetate (200 ml). The reaction mixture was washed sequentially with water (50 ml) and an aqueous solution of saturated sodium bicarbonate (50 ml, twice). And then, the mixture was washed with saturated brine (50 ml) and dried over sodium sulfate. The solid matter was filtered off, and the solution was concentrated to obtain a light yellow compound (6-b). The crude product was recrystallized with n-hexane/ethyl acetate (3/7) to isolate a compound (6-b) (an isomer mixture) at a yield of 76% as a white solid.

### (Example 21)

The compound (6-b) (1.0 MR, 1.0 g, 1.70 mmol) was dissolved in tetrahydrofuran (50 ml), and the mixture was cooled to 0°C. Next, a solution of sodium hydroxide (2.4 MR, 0.163 g, 4.10 mmol) in water (5 ml) was slowly added dropwise thereto at the same temperature. The reaction mixture was allowed to naturally warm to room temperature and stirred for 20 hours. At this point, the reaction progress was checked by HPLC, and the reaction was quenched and a post treatment was performed. The resulting product was analyzed by HPLC. It was found that the product contained 83% (LC area percentage) of the desired compound (7-b) and 3.5% (LC area percentage) of the starting compound (6-b).

Analysis data:
¹H-NMR (500MHz, CDCl₃, δ): 7.70-7.74 (4H, m), 7.33-7.36 (4H, m), 5.21-5.26 (1H, m), 4.25-4.38 (1H, dd, J:5.7, 14.1Hz), 4.02-4.05 (1H, dd, J:6.8Hz), 3.61-3.73 (2H, m), 3.57 (1H, br s), 3.11-3.17 (1H, m), 2.63-2.67 (1H, m), 2.46 (3H, s), 2.45 (3H, s), 2.21 (3H, sx2), 2.08-2.16 (1H, m), 1.68-1.91 (1H, m), 1.31 (3H, sx2), 1.23 (3H, s x2), 1.06-0.99 (3H, sx2). FD-MS: 546 (M+H).

### INDUSTRIAL APPLICABILITY

According to the present invention, sulfone compounds which are important intermediates for carotenoids can be produced by commercially advantageous processes.

## Claims

1. A process for producing a compound represented by the following formula (1) in which Z is a hydrogen atom: 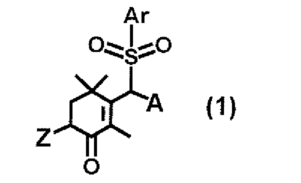 [wherein A is a hydrogen atom or a group represented by the formula (2): 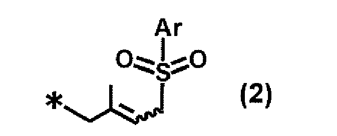 (wherein Ar is an aryl group optionally having 1 to 3 substituents, the wavy line means that the steric relation to the double bond which the wavy line is bound to is of E-form, a Z-form or a mixture of E/Z, and * shows a bonding site) and Ar means the same as defined above], comprising:
a first step of reacting a compound represented by the formula (3): 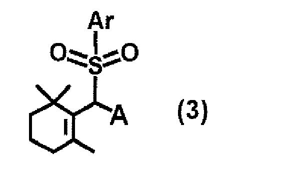 (wherein A and Ar mean the same as defined above) with a chlorate or a bromate; and
a second step of reacting the reaction liquid obtained in the first step with either of reaction reagents: HX and X₂ wherein X is a halogen atom to give a compound represented by the formula (4): 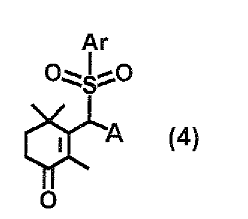 (wherein A and Ar mean the same as defined above).

2. The process of claim 1, wherein the second step is performed at pH 5 or less.

3. The process of claim 1 or 2, wherein the chlorate or the bromate is an alkali metal salt.

4. The process of claim 3, wherein the alkali metal salt of chloric acid or bromic acid is sodium chlorate or sodium bromate.

5. The process of any one of claims 1 to 4, wherein the reaction reagent used in the second step is HX wherein X is a bromine atom.

6. The process of any one of claims 1 to 4, wherein the reaction reagent used in the second step is X₂ wherein X is an iodine atom.

7. The process of any one of claims 1 to 6, wherein Ar is a 4-methylphenyl group.

8. A process for producing a compound represented by the following formula (1) in which Z is a halogen atom: 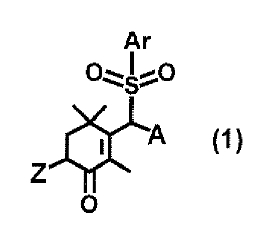 [wherein A is a hydrogen atom or a group represented by the formula (2): 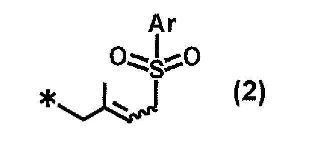 (wherein Ar is an aryl group optionally having 1 to 3 substituents, the wavy line means that the steric relation to the double bond which the wavy line is bound to is of E-form, a Z-form or a mixture of E/Z, and * shows a bonding site) and Ar means the same as defined above], comprising the step of:
reacting a compound represented by the formula (4): 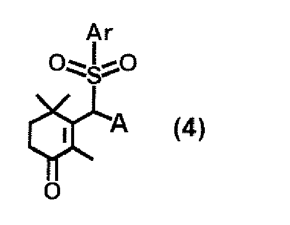 (wherein A and Ar mean the same as defined above) with a halogenating agent to give a compound represented by the formula (5): 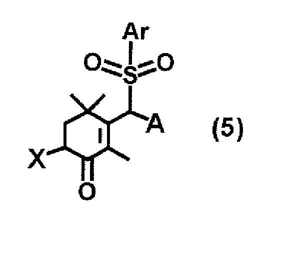 (wherein X is a halogen atom, and A and Ar mean the same as defined above).

9. The process of claim 8, wherein X is a bromine atom, and the halogenating agent is bromine, N-bromosuccinimide or 1,3-dibromo-5,5-dimethylhydantoin.

10. The process of claim 8 or 9, wherein Ar is a 4-methylphenyl group.

11. A process for producing a compound represented by the following formula (1) in which Z is an alkoxycarbonyl group: 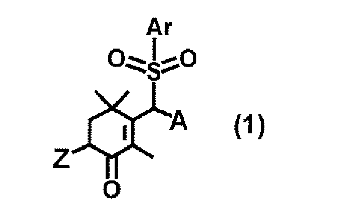 [wherein A is a hydrogen atom or a group represented by the formula (2): 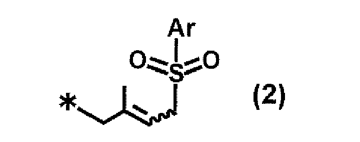 (wherein Ar is an aryl group optionally having 1 to 3 substituents, the wavy line means that the steric relation to the double bond which the wavy line is bound to is of E-form, a Z-form or a mixture of E/Z, and * shows a bonding site) and Ar means the same as defined above], comprising the step of:
reacting a compound represented by the formula (4): 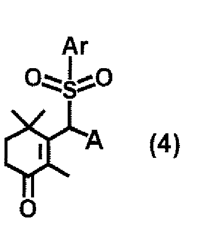 (wherein A and Ar mean the same as defined above) with a compound represented by M(OCOR)ₙ (wherein M is a metal atom, R is an alkyl group having 1 to 4 carbon atoms, and n is an integer of 2 to 4), to give a compound represented by the formula (6): 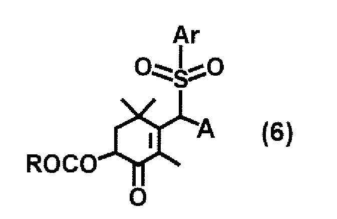 (wherein A, Ar and R mean the same as defined above).

12. The process of claim 11, wherein R is a methyl group, M is manganese, and n is 3.

13. The process of claim 11 or 12, wherein Ar is a 4-methylphenyl group.

14. A process for producing a compound represented by the following formula (1) in which Z is a hydroxyl group: 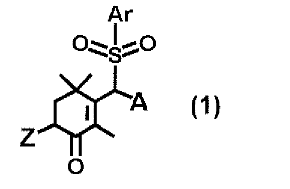 [wherein A is a hydrogen atom or a group represented by the formula (2): 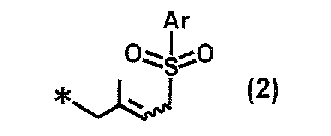 (wherein Ar is an aryl group optionally having 1 to 3 substituents, the wavy line means that the steric relation to the double bond which the wavy line is bound to is of E-form, a Z-form or a mixture of E/Z, and * shows a bonding site) and Ar means the same as defined above], comprising the step of:
reacting a compound represented by the formula (6): 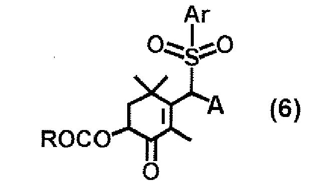 (wherein R is an alkyl group having 1 to 4 carbon atoms, and A and Ar mean the same as defined above) with an inorganic base to give a compound represented by the formula (7): 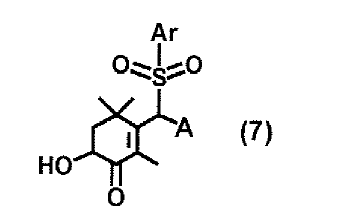 (wherein A and Ar mean the same as defined above).

15. The process of claim 14, wherein the inorganic base is a hydroxide, a carbonate or a hydrogen carbonate of an alkali metal or an alkaline earth metal.

16. The process of claim 14 or 15, wherein Ar is a 4-methylphenyl group.

17. A compound represented by the formula (1): 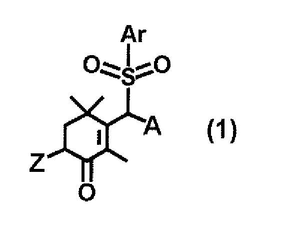 [wherein Z is a hydrogen atom, a halogen atom, an alkoxycarbonyl group having 1 to 4 carbon atoms, or a hydroxyl group; A is a hydrogen atom or a group represented by the formula (2): 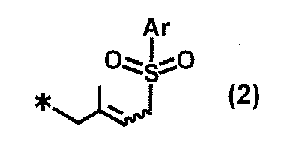 (wherein Ar is an aryl group optionally having 1 to 3 substituents, the wavy line means that the steric relation to the double bond which the wavy line is bound to is of E-form, a Z-form or a mixture of E/Z, and * shows a bonding site) and Ar means the same as defined above,
provided that when A is a hydrogen atom, Z is not a halogen atom and a hydroxyl group].

18. The compound of claim 17, wherein Ar is a 4-methylphenyl group.

19. The compound of claim 17 or 18, wherein Z is a methoxycarbonyl group.
